Europäisches Patentamt

European Patent Office

Office européen des brevets

(19)

(11) Veröffentlichungsnummer: **0 001 860**
**B1**

(12) **EUROPÄISCHE PATENTSCHRIFT**

(45) Veröffentlichungstag der Patentschrift:
05.05.82

(51) Int. Cl.³: **A 61 K 7/06**

(21) Anmeldenummer: 78200271.1

(22) Anmeldetag: 30.10.78

(54) **Verwendung von Weinhefeöl oder seinen wesentlichen Bestandteilen als Haarpflege-,insbesondere Haarwuchsmittel.**

(30) Priorität: 03.11.77 DE 2749166

(43) Veröffentlichungstag der Anmeldung:
16.05.79 Patentblatt 79/10

(45) Bekanntmachung des Hinweises auf die Patenterteilung:
05.05.82 Patentblatt 82/18

(84) Benannte Vertragsstaaten:
**BE CH DE FR GB LU NL SE**

(56) Entgegenhaltungen:
**CH-A-179254**
**DE-C-464745**

**HANDBUCH DER KOSMETIKA UND RIECHSTOFFE,**
**Band II, 1969, Dr.A.Flüthig Verlag, Heidelberg,**
**H. JANISTIJN «Die Parfümerie in der Kosmetik»**

(73) Patentinhaber: **Kellersch, Josef, Sallerring 9,**
**D-5000 Köln 1 (DE)**

(72) Erfinder: **Kellersch, Josef, Sallerring 9, D-5000 Köln 1 (DE)**

(74)Vertreter: **Eggert, Hans-Gunther, Dr.,**
**Raederscheidtstrasse 1, D-5000 Köln 41 (DE)**

EP 0 001 860 B1

## Verwendung von Weinhefeöl oder seinen wesentlichen Bestandteilen als Haarpflege-, insbesondere Haarwuchsmittel

Die Erfindung betrifft die Verwendung von Weinhefeöl oder seinen wesentlichen Bestandteilen als Mittel gegen Haarausfall bzw. zur Forderung des Haarwuchses.

In Janistyn's Handbuch der Kosmetika und Riechstoffe, II. Band ist auf Seite 29 die Verwendung von Weinhefeöl unter anderem als Shampooparfüm beschrieben. Die haarwuchsfordernde Wirkung des Weinhefeöls kommt bei der Verwenes ist in einer viel zu geringen Menge anwesend und wird bei der Kopfwäsche viel zu schnell abgewaschen.

Aus der DE-PS 464 745 ist ein Verfahren zur Herstellung eines Haarmittels bekannt, bei dem Rautenblätter in Gegenwart von Weinhefe einem Gärungsprozess unterworfen werden, worauf die Flüssigkeit abgepresst, filtriert und mit Spiritus oder Parfüm versetzt wird.

In der CH-PS 179 254 ist ein Verfahren zur Herstellung von Haarcreme beschrieben, bei dem «eau-de-vie de lie», d. h. Tresterschnaps verwendet wird.

Weinhefeöl wird in der Fachliteratur als Oleum Vini, Cognacöl, Weinbeeröl, Weinbrandöl, Weinol oder Önantäther bezeichnet. Man gewinnt das Weinhefeöl aus den nach dem Abziehen des klar gewonnenen Weins in Fässern verbleibenden Rückständen oder nach dem Abpressen des Weins zurückbleibenden Hefekuchen durch einfache Destillation mit Wasser, sofern in dem Hefetrub nicht schon genügend Wasser vorhanden war. Nach Abdestillieren des Alkohols geht mit Wasserdampf das schwer flüchtige Weinöl über, das in der Vorlage auf dem alkoholhaltigen Wasser schwimmt. Aus der wässrigen Phase des Destillats können durch Extraktion oder Aussalzen noch weitere Mengen Öl gewonnen werden. Im rohen Zustand ist Weinhefeöl ein durch Kupferverbindungen noch grün gefärbtes, raffiniert ein farbloses leicht bewegliches ätherisches Öl von betäubendem, in unverdünntem Zustand eher unangenehmen Geruch. Das rohe Weinol kann durch Ausschütteln mit Weinsäurelösung von Kupferverbindungen und durch Behandeln mit Sodalösung von freien Fettsäuren befreit und anschliessend rektifiziert werden, wobei noch ein Teil der nur schwach riechenden Ester höherer Fettsäuren zurückgehalten wird.

Weinhefeöl hat im allgemeinen einen Siedepunkt zwischen 225 und 230° C. Es besteht überwiegend aus Caprinsäureathyl- und Caprinsaur amylester sowie den Athyl- und Amylestern der Capryl-Pelargon- und Laurinsaure.

Es hat sich gezeigt, dass Weinhefeöl von der Kopfhaut sehr gut und ohne Schaden aufgenommen wird. Um die Anwendung zu erleichtern kann es mit wässrigen alkoholischen Losunge verdünnt oder mit Duftstoffen versehen werden Es genügt eine sehr kurzzeitige Einwirkung, um selbst hartnäckigen Haarausfall zu stoppen un den Haarwuchs zu fördern.

Es ist anzunehmen, dass die überraschend Wirkung des Weinhefeöls auf einen oder mehre ren der darin enthaltenen atherischen Öle zurücl zuführen ist, so dass diese unter Umstanden auc für sich allein oder in einem anderen Mischungs verhältnis als es im naturlichen Weinol vorlieg für den erfindungsgemässen Zweck eingeset werden können. Auch künstliches Weinhefec oder Cognacol dürfte daher für die Zwecke de Erfindung brauchbar sein.

### Patentanspruch

Verwendung von Weinhefeol oder seinen we sentlichen Bestandteilen, Caprinsaureathyl- un Caprinsäureamylester, Athyl- und Amylester de Capryl-, Pelargon- und Laurinsaure als Mittel ge gen Haarausfall bzw. zur Forderung des Haar wuchses.

### Claim

1. The use of wine yeast oil or its essential constituents, ethyl caprate and amyl caprate, ethyl and amyl esters of caprylic, pelargonic and lauric acids as a remedy for hair loss or to promote hair growth.

### Revendication

Utilisation de l'essence de lie de vin ou de ses constituants essentiels, le caprate d'ethyle et le caprate d'amyle, les éthers ethyliques et amyliques de l'acide caprylique, de l'acide pelargonique et de l'acide laurique en tant qu'agents protegeant contre la chute des cheveux ou favorisant la croissance des cheveux.